# EUROPEAN PATENT SPECIFICATION

(11) **EP 0 344 270 B1**
(45) Date of publication and mention of the grant of the patent: **30.11.1994**
(21) Application number: 89900419.6
(22) Date of filing: 18.10.1988
(51) Int. Cl.: C12Q 1/68, G01N 33/553, C02F 1/48

(54) **PROCESS FOR PRODUCING MAGNETICALLY RESPONSIVE POLYMER PARTICLES AND APPLICATION THEREOF**
VERFAHREN ZUR HERSTELLUNG MAGNETISCH EMPFINDLICHER POLYMERTEILCHEN UND DEREN ANWENDUNG
PROCEDE DE PRODUCTION DE PARTICULES POLYMERES A SENSIBILITE MAGNETIQUE ET LEURS APPLICATIONS

(30) Priority: 26.10.1987 US 113294
(43) Date of publication of application: 06.12.1989
(73) Proprietor: Baxter Diagnostics Inc., Deerfield, Illinois 60015-4633 (US)
(72) Inventor: WANG, Chao-Huei, J., Gurnee, IL 60031 (US); SHAH, Dinesh, O., Vernon Hills, IL 60061 (US)
(74) Representative: Bassett, Richard Simon
(86) International application number: US8803666
(87) International publication number: WO8904373

(56) References cited:
- EP-A- 0 125 995
- EP-A- 0 234 083
- WO-A-86/05815
- WO-A-87/02063
- US-A- 3 560 378
- US-A- 4 070 246
- US-A- 4 177 253
- US-A- 4 219 335
- US-A- 4 230 685
- US-A- 4 272 510
- US-A- 4 285 819
- US-A- 4 382 982
- US-A- 4 438 239
- US-A- 4 490 436
- US-A- 4 496 658
- HE LANCET I, no. 8368, January 1984, London (GB); J.G. TRELEAVEN et al., pp.70-73 #
- S.S. DAVIS ET AL. " Microspheres and drug therapy. Immunological and medical aspects". 1984, ELSEVIER SCIENCE PUBLISHERS. Amsterdam (NL), pages 365-382.

## Description

### Field of the Invention

This invention relates to a process to make magnetically responsive polymer particles and their use in immunoassays, biomedical and industrial applications.

### Background of the Invention

Many biological techniques, such as immunoassays, affinity purification, etc., require the separation of bound from free fractions. Magnetic particles have been used to facilitate the desired separation.

Magnetic particles have been formed from a variety of particulate and magnetic matter, using a variety of processes, having different characteristics. For example, Ikeda et al. US-A-4,582,622, discloses a magnetic particle comprised of gelatin, water-soluble polysaccharide, sodium phosphate and ferromagnetic substances; US-A-4,628,037 and US-A-4,554,088 disclose magnetic particles comprised of a magnetic metal oxide core surrounded by a coat of polymeric silane; US-A-4,452,773 discloses discrete colloidal sized particles having a core of ferromagnetic iron oxide (Fe₃0₄) which is coated with a water-soluble polysaccharide or a derivative thereof having functional groups; and Mansfield US-A-4,297,337 discloses magnetic glass- or crystal-containing material as a particulate carrier. US-A-4438239 and EP-A-0234083 also disclose carriers comprising particles of metal oxide and polymer combinations.

### Summary of the Invention

The present invention provides monodispersed magnetic particles as claimed in Claim 1 and a process of making a magnetically responsive polymer particle as claimed in Claim 15.

Magnetically responsive polymer particles are hereinafter referred to as magnetic particles. The particles of the invention may have an average size from about 1 to 100 microns in diameter regardless of shape and composition. The magnetic particles of this invention may be prepared by first producing magnetically responsive metal oxide, hereinafter referred to as metal oxide, with average size of about 1 micron or less and then coating a polymeric core particle with a layer of polymer containing metal oxide. The surface of these magnetic particles can be coated further with another layer of polymer or functionalized polymer to provide the desired surface characteristics.

The magnetic particles produced by the present invention are monodispersed in size with rough surface and may have a magnetic metal oxide content of from about 5% to 50%, preferably from 10% to 25%. Particles with these characteristics have been found to be useful in immunoassays and a wide variety of biomedical applications. These magnetic particles can be used for passive or covalent coupling of biological material such as antigens, antibodies, enzymes or DNA/RNA and used as solid phase for various types of immunoassays, DNA/RNA hybridization assays, affinity purification, cell separation and other biomedical applications. The magnetic particles can also be used for industrial application such as the treatment of industrial waste.

### Objectives and Advantages

It is the objective of this invention to:
Develop a process of producing magnetically responsive polymer particles easily from readily available polymer particles.

Develop a process of producing magnetically responsive polymer particles with moderate sedimentation and fast magnetic separation.

Develop a process of producing magnetically responsive polymer particles with various surface charges, and functional groups for passive adsorption or covalent coupling of biological material.

Develop medical, biological, diagnostic and industrial applications using these magnetically responsive polymer particles.

The advantages of this invention include:
A wide variety of polymeric core particles with size from about 1 to 100 microns can easily be transformed to magnetically responsive particles.

The metal oxide content can be varied according to the applications.

The surface can be derivatized into a wide variety of functional groups for covalent coupling.

A wide variety of monomer can be used for the final coating to provide different surface characteristics of the resulting polymer.

Both crosslinked and noncrosslinked magnetically responsive polymer particles can be produced.

Monodispersed magnetically responsive polymer particles can be produced.

### Detailed Description of the Invention

The magnetic particles of this invention may be prepared by first producing metal oxide with average size of about 1 micron or less. The metal oxide is produced by heating and precipitating a mixture of divalent and trivalent metal salt, preferably a mixture of ferrous and ferric sulfate or chloride with sodium hydroxide solution. The molar ratio of divalent to trivalent metal salt can be varied from 0.5 to 2.0, preferably 0.5 to 1.0, to obtain the desirable size and magnetic characteristics of metal oxide. It is observed that the molar ratio of divalent to trivalent metal salt affects the size of the metal oxide: the smaller the molar ratio of divalent to trivalent metal salt, the smaller the size of metal oxide. The molar ratio of divalent to trivalent metal salt also affects the color of the resulting magnetic particles: the smaller the molar ratio, the lighter the brownish color of the resulting magnetic particles. Preferably, the metal oxide is either superparamagnetic or paramagnetic although ferromagnetic metal oxide can also be used, provided centrifugation instead of magnetic separation is used during the clean up. Other divalent transition metal salts such as manganese, magnesium, cobalt, nickel, zinc, and copper salts may be substituted for ferrous salt.

After the metal oxide has been precipitated, it is washed several times with centrifugation at 250xg until the supernatant is neutral in pH. The metal oxide is resuspended in deionized water and mechanically stirred at high speed to break down the aggregate of metal oxide crystals. Further centrifugation at 250xg will not pellet all of the metal oxide. The supernatant which contain smaller size metal oxide crystals is collected and the pellet is resuspended in deionized water. This process is repeated for at least three times or until most of metal oxide can no longer be pelleted at 250xg. The metal oxide obtained this way usually has size less than 2.0 micron. Low speed centrifugation at 100xg to remove largers crystals will reduce the size to less than 0.8 micron.

The metal oxide with average size of 1.0 micron or less is mixed with monomer and coated onto the polymeric core particles, preferably polystyrene particles, with size of 1 to 100 microns in the presence of initiator. Addition of a small quantity of emulsifier will help prevent the particles from agglomerating. The magnetic particles are then coated with a protective layer of polymer, preferably polystyrene, to prevent the metal oxide from falling off. If functionalized magnetic particles are desired the magnetic particles can be coated further with another layer of functionalized polymer to provide functional groups such as carboxyl, amino or hydroxyl for covalent coupling of biological material.

The magnetic particles prepared according to this invention can be illustrated in Figure I, where A represents the core particle, B represents the metal oxide/polymer coating, C represents the protective polymer coating and D represents the functionalized polymer coating. Figure II shows the transmission electron micrograph of 0.08 to 0.1 micron slice of a magnetic particle prepared according to this invention. Figure III shows a scanning electron micrograph of 6.8 micron magnetic particles, prepared according to this invention. Figure III a is at 1000x and Figure III b is at 5000x magnification.

The polymeric core particles useful in this invention may be of any polymer which can be obtained as a dispersion of small particles and which can absorb a monomer thereby causing the metal oxide and monomer mixture to coat onto the surface of the core particles. The core particles may be of any size and shape, preferably of 1 to 100 microns in size and spherical in shape. When monodispersed core particles are used the resulting magnetic particles will also be monodispersed in size. The core particles may be obtained by emulsion polymerization, suspension polymerization or other means of polymerization with or without a crosslinking agent such as divinyl benzene or the like. Among the monomers which can be used to prepare core particles are styrene, methyl methacrylate, vinyltoluene, divinyl benzene, glycidyl methacrylate and other olefinic monomers. A mixture of the monomers can also be used. The monomer used for magnetic metal oxide coating or protective coating may or may not be the same type as the core particles. The weight ratio of monomer used for metal oxide coating to core particles may be from 0.1 to 12, preferably from 0.2 to 6, depending upon the thickness of metal oxide/polymer layer desired. When the metal oxide prepared from a mixture of ferrous and ferric salts is used for coating it is preferred to use a monomer to core particle weight ratio of about 0.1 to 0.5. However when the metal oxide prepared from a mixture of manganese (II) and ferric salts is used for coating the weight ratio of monomer to core particles may be from 0.1 to 12. As a result when cross-linked magnetic particles which are inert to common organic solvent are desired, it is preferred to use the metal oxide prepared from a mixture of manganese (II) and ferric salts with monomer containing 2% to 10%, preferably 8% to 10% by weight of crosslinking agent and a monomer to core particle weight ratio of 3 to 12, preferably 4 to 6. When lower monomer to core particle weight ratio (i.e. 0.1 to 0.5) is used during the metal oxide/polymer coating it is preferred to overcoat the resulting magnetic particles with a protective layer of polymer coating to further adhere the metal oxide to the surface of the magnetic particles. However, when higher monomer to core particle ratio (i.e. 3 to 12) is used no protective polymer coating is necessary. The polymerization temperature may be from 50°C to 90°C, preferably 55°C to 65°C. The polymerization initiator may either be water soluble such as potassium persulfate and the like or water insoluble such as benzoyl peroxide and the like. Other means of polymerization initiation such as radiation, ionization or the like may also be used. It is found unexpectly that magnetic particles can be produced without using any emulsifier when the metal oxide prepared from a mixture of manganese (II) and ferric salts is used for coating. However, a small amount of emulsifier such as sodium dodecylsulfate, Aerosol 22, Tween 20 or Nonidet P-40 (NP 40) is found to be useful in preventing the particles from extensive aggregation during the metal oxide/polymer coating when the metal oxide prepared from a mixture of ferrous and ferric salts is used for coating. Other emulsifiers with the same capability may also be used. The magnetic metal oxide content can be varied from 5% to 50%, preferably from 10% to 25% by using different amount of metal oxide during the metal oxide/polymer coating. Mutiple metal oxide/polymer coatings can also be employed to increase the metal oxide content. Other ingredients commonly used in polymerization may also be added as long as magnetic particles with desirable characteristics can be obtained. The ingredients for metal oxide/polymer coating may be added all at once at the beginning of metal oxide/polymer coating process or added stepwise. When the metal oxide prepared from a mixture of ferrous and ferric salt is used, it is preferred to add the ingredients stepwise. The ingredients may be mixed by mechanic stirring, tumbling or other means of agitation under vacuum or inert gas such as argon. The functional groups can be incorporated onto the surface of the magnetic particles by either using a mixture of monomer and functionalized monomer during the metal oxide/polymer coating or overcoating the magnetic particles with a thin layer of functionalized monomer at the end. The functionalized monomer used may be selected from one or a mixture of the following: 2-hyroxyethyl methacrylate, 2-aminoethyl methacrylate, trimethylammoniumethyl methacrylate methosulfate, dimethylaminoethyl methacrylate, methacrylic acid, undecylenic acid, methyl propene sulfonic acid, undecylenyl alcohol, oleyl amine, glycidyl methacrylate, acrolein, glutaraldehyde and the like. The magnetic particles can also be overcoated with a layer of different polymer than the one used for metal oxide/polymer coating or protective coating to take up the surface characteristics of that polymer.

### Applications of Magnetic Particles

The uses of a wide variety of magnetic particles as solid phase for various applications such as fluorescence immunoassays, radioimmunoassays, enzyme immunoassays, cell separations, enzyme immobilizations and affinity purificatins have been reviewed in literature as examplified by the following articles: Hirschbein et al, Chemical Technology, March 1982, 172-179 (1982); Pourfarzaneh, The Ligand Quarterly, 5(1): 41-47 (1982); Halling and Dunnill, Enzyme Microbe Technology, 2: 2-10 (1980); Mosbach and Anderson, Nature, 270: 259-261 (1977); Guesdon et al, J. Allergy Clinical immunology, 61(1), 23-27 (1978). Some applications have also been disclosed in the U.S. Patent Nos. 4,152,210 and 4,343,901 for enzyme immobilizatins; U.S. Patent Nos. 3,970,518, 4,230,685, and 4,267,2343 for cell separations; U.S. Patent Nos. 4,554,088, 4,628,037, and 3,933,997 for immunoassays.

Some magnetic particles may be useful in one application, but not in another application. For example, the magnetic particles disclosed in U.S. Patent No. 4,554,088 and 4,628,037, which comprise a superparamagnetic metal oxide core generally surrounded by a coat of polymeric silane, may be useful in immunoassay and affinity purification, due to the large surface area and slower settling rate, but are not suitable in cell separation application such as bone marrow purging. Due to the small size of the magnetic particles, disclosed in these two patents, it is very difficult to remove all of the magnetic particles from the cell suspension effectively. Moreover, the nonspecific binding of smaller magnetic particles to normal cells would be much higher. In using magnetic particles for bone marrow purging, the magnetic particles are coated with antibody, such as sheep anti-mouse IgG, and the bone marrow is treated with a mixture of several monoclonal antibodies against the cancer cell surface antigens. The magnetic particles will bind only to the cancer cells and cause them to be separated from normal cells by passing them through a strong magnetic field. The cleansed cells are then put back into the patient.

By using the processes of this invention magnetic particles can be optimized in terms of size, surface area, metal oxide content and surface characteristics for a wide variety of biomedical applications. The magnetic particles produced by this invention can be used as solid phase for enzyme immunoassay, fluorescence immunoassay, radioimmunoassay, DNA/RNA hybridization assay, and other diagnostic applications. Immunoassays can be performed by using various configurations such as sandwich assays and competitive binding assays, etc., which are obvious to those skilled in the art. The DNA/RNA hybridization can also be performed by using various configurations such as solid phase hybridization or liquid phase hybridization. In solid phase hybridization configuration a DNA or RNA probe (cather probe) is immobilized on the magnetic particle first. The immobilized cather probe is then used to hybridize with complimentary strand of DNA from the sample (sample DNA). Finally another probe (signal probe) which is labelled with fluorescent, radioactive or enzyme tracer and capable of hybridizing with another part of the sample DNA is used for signal generation. In liquid phase hybridization configuration the cather probe and signal probe are allowed to hybridize with the sample DNA in the liquid phase first and then immobilized to the magnetic particles.

Alternatively, the signal probe can also be labelled with one or several biotin groups and the signal is detected by binding the biotin groups with avidin labelled fluorescent, radioactive or enzymatic tracer to enhance the sensitivity of the assay.

The immunoassays and DNA/RNA hybridization assays can be used to measure a wide variety of compounds such as enzymes, drugs, hormones, antibodies, peptides, vitamins, DNA, RNA, nucleic acids, nucleotides, oligonucleotides, biological cells, viral antigens, antibodies, haptens and carbohydrates in biological samples.

The magnetic particles produced by this invention can also be used for affinity purification, cell separation, enzyme immobilization and other biomedical applications. In cell separation the magnetic particles are used to either remove unwanted cells (negative selection) or enrich the wanted cells (positive selection) through immunological reactions or nonimmunological reactions. This principle can be used to remove cancer cells from bone marrow (bone marrow purging), purify cell populations through either positive or negative selection for tissue culture and perform various cellular immunoassays etc. In affinity purification the magnetic particles are used in place of conventinal solid phase such as polyacrylamide gels, sepharose gels or other cellulose beads to purify a wide variety of biological materials such as antibodies, antigens, enzymes. inhibitors, cofactors, single stranded DNA, binding proteins, haptens and carbohydrates etc. In another application similar to the affinity purification, the magnetic particles can be used to cross adsorb and remove unwanted protein components from the antisera or clinical samples. In enzyme immobilization the enzyme is immobilize onto the magnetic particles through various means of coupling so as to preserve the enzyme activity and to permit the reuse of immobilized enzyme. The magnetic particles with immobilized enzyme can be used to replace other solid phases such as glass beads, controled pore glass, silica gels and cellulose beads etc., which are commonly used in immobilized enzyme systems to produce a wide variety of materials such as carbohydrates, amino acids, and proteins, etc.

The magnetic particles produced by this invention can be used for industrial applications like the treatment of industrial waste, to remove harmful chemicals, i.e. organic or inorganic solvents from industrial material.

These applications are all facilitated by the ease of separation, fast reaction rate and large surface area common to most of magnetic particles. The following examples are provided to further illustrate the versatility and advantages of this invention.

### General Procedures for the Preparation of Metal Oxide

### Example 1

In a three-necked round bottom flask equipped with mechanical stirrer, condenser, thermometer, dropping funnel and heating mantle was placed a mixture containing 0.361 mol of ferrous sulfate and 0.369 mol of ferric sulfate (Fe⁺⁺/Fe⁺⁺⁺ ratio = 1) in 400 ml of deionized water. The mixture was heated to 85 to 90 °C with stirring and added dropwise 850 ml of 6 N sodium hydroxide over a period of 90 minutes. The mixture was stirred at 85 to 90 °C for one more hour and cooled to room temperature. The metal oxide precipitates were centrifuged at 250xg for 10 minutes. The clear supernatant was decanted and the pellet was resuspended in 900 ml of deionized water using mechanical stirrer. This cleaning process was repeated six times or until the supernatant was almost neutral in pH. The supernatant was decanted and resuspended in 200 ml of deionized water. Further centrifugation at 250xg will not pellet all of the metal oxide precipitates. The supernatant which contained smaller size metal oxide crystals was collected and the pellet was resuspended in 200 ml of deionized water. This process was repeated for at least three times or until most of metal oxide can no longer be pelleted at 250xg. The metal oxide obtained this way usually has size less than 2.0 micron. The combined metal oxide suspension was centrifuged at 100xg for 10 minutes. The supernatant was collected to give 800 ml of 8.6 % w/v magnetic metal oxide suspension having the size less than 0.8 microns.

### Example 2

Same procedures as described in Example 1 were followed except 0.235 mol of ferrous sulfate, 0.297 mol of ferric sulfate (Fe⁺⁺/Fe⁺⁺⁺ ratio = 0.79) in 400 ml of deionized water and 480 ml of 6 N sodium hydroxide were used to give 2000 ml of 2.86 % w/v suspension of magnetic metal oxide.

### Example 3

Same procedures as described in Example 1 were followed except 0.178 mol of ferrous sulfate, 0.298 mol of ferric sulfate (Fe⁺⁺/Fe⁺⁺⁺ ratio = 0.59) in 400 ml of deionized water and 520 ml of 6 N sodium hydroxide were used to give 1500 ml of 2.98 % w/v suspension of magnetic metal oxide.

### Example 4

Same procedures as described in Example 1 were followed except 0.15 mol of ferrous sulfate, 0.276 mol of ferric sulfate (Fe⁺⁺/Fe⁺⁺⁺ ratio = 0.54) in 400 ml of deionized water and 520 ml of 6 N sodium hydroxide were used to give 700 ml of 6.88 % w/v suspension of magnetic metal oxide.

### Example 5

Same procedures as described in Example 1 were followed except 0.116 mol of manganese sulfate, 0.146 mol of ferric sulfate (Mn⁺⁺/Fe⁺⁺⁺ ratio = 0.79) in 225 ml of deionized water and 240 ml of 6 N sodium hydroxide were used to give 1700 ml of 1.8 % w/v suspension of magnetic metal oxide.

### Preparation of Magnetic Particles

### Example 6

A mixture containing 600 ml of deionized water, 6 ml of styrene and 80 ml of 8.6 % w/v magnetic metal oxide prepared as described in Example 1, was placed in a sealed bottle. The bottle was evacuated and rotated at about 60 rpm in a 55 °C oven for one hour. To the mixture were added 12 g of potassium persulfate and 850 ml of 5 % w/v, 4.0 micron polystrene particles. The bottle was resealed, evacuated and rotated for one hour and added 50 ml of 2 % sodium dodecylsulfate. After five more hours 6 ml of styrene and 10 g of potassium persulfate were added to the mixture. The mixture was rotated for another fifteen hours, filtered through two layers of cheese cloth, separated magnetically and washed several times with deionized water until the supernatant was clear. The resulting magnetic particles were resuspended to 1.6 l with deionized water to give a 2.5 % w/v suspension with about 11 % magnetic metal oxide content and 4.3 micron average size.

### Example 7

The magnetic particles, 1.6 l of 2.5 % w/v, prepared as described in Example 6, were carboxylated by adding 1 g of sodium dodecylsulfate, 10 g of potassium persulfate and a solution containing 0.98 ml of undecylenic acid and 0.02 ml of divinyl banzene in 4 ml of methanol. The mixture was placed in a sealed bottle, evacuated and rotated at about 60 rpm in a 55 °C oven for 5 hours. The resulting carboxyl magnetic particles were separated magnetically and washed several times with deionized water until the supernatant was clear. The carboxyl magnetic particles were resuspended to 680 ml with deionized water to give a 5.8 % w/v suspension with about 11 % magnetic metal oxide content and 4.3 micron average size.

### Example 8

A mixture containing 600 ml of deionized water, 6 ml of styrene and 80 ml of 8.6 % w/v magnetic metal oxide prepared as described in Exmaple 1, was placed in a sealed bottle. The bottle was evacuated and rotated at about 60 rpm in a 55 °C oven for one hour. To the mixture were added 12 g of potassium persulfate and 850 ml of 4.78 % w/v, 6.1 micron polystrene particles. The bottle was resealed, evacuated, rotated for five hours and added 6 ml of styrene and 10 g of potassium persulfate. The mixture was rotated for another fifteen hours, filtered through two layers of cheese cloth, separated magnetically and washed several times with deionized water until the supernatant was clear. The resulting magnetic particles were resuspended to 1.5 l with deionized water and carboxylated by adding 1 g of sodium dodecylsulfate, 10 g of potassium persulfate and a solution containing 0.98 ml of undecylenic acid and 0.02 ml of divinyl benzene in 4 ml of methanol. The mixture was placed in a sealed bottle, evacuated and rotated at about 60 rpm in a 55 °C oven for 5 hours. The resulting carboxyl magnetic particles were separated magnetically and washed several times with deionized water until the supernatant was clear. The carboxyl magnetic particles were resuspended to 800 ml with deionized water to give a 4.3 % suspension with about 11.6 % magnetic metal oxide content and 6.8 micron average size.

### Example 9

A mixture containing 600 ml of deionized water, 6 ml of styrene and 60 ml of 8.6 % w/v magnetic metal oxide prepared as described in Example 1, was placed in a three-necked round bottom flask and stirred at 67 °C for one hour under argon. To the mixture were added 12 g of potassium persulfate and 470 ml of 5 % w/v, 2.7 micron polystrene particles. The mixture was stirred at 67 °C for one hour and added 30 ml of 2 % sodium dodecylsulfate. After stirring at 67 °C under argon for five more hours 6 ml of styrene and 6 g of potassium persulfate were added to the mixture. The mixture was stirred at 67 °C under argon for another fifteen hours, filtered through two layers of cheese cloth, separated magnetically and washed several times with deionized water until the supernatant was clear. The resulting magnetic particles were resuspended to 900 ml with deionized water and carboxylated by adding 0.6 g of sodium dodecylsulfate, 10 g of potassium persulfate and a solution containing 0.598 ml of undecylenic acid and 0.012 ml of divinyl benzene in 2.4 ml of methanol. The mixture was placed in a sealed bottle, evacuated and rotated at about 60 rpm in a 55 °C oven for 5 hours. The resulting carboxyl magnetic particles were separated magnetically and washed several times with deionized water until the supernatant was clear. The carboxyl magnetic particles were resuspended to 500 ml to give a 6.5 % w/v suspension with about 14 % magnetic metal oxide content and 4.0 micron average size.

### Example 10

A mixture containing 600 ml of deionized water, 6 ml of styrene and 60 ml of 8.6 % w/v magnetic metal oxide prepared as described in Example 1, was placed in a sealed bottle. The bottle was evacuated and rotated at about 60 rpm in a 55 °C oven for one hour. To the mixture were added 12 g of potassium persulfate and 470 ml of 5 % w/v, 2.7 micron polystyrene particles. The bottle was resealed, evacuated and rotated for one hour and added 30 ml of 2 % sodium dodecylsulfate. After five more hours 6 ml of styrene and 10 g of potassium persulfate were added to the mixture. The mixture was rotated for another fifteen hours, filtered through two layers of cheese cloth, separated magnetically and washed several times with deionized water until the supernatant was clear. The resulting magnetic particles were resuspended to 500 ml with deionized water to give a 6.8 % w/v suspension with about 14 % magnetic metal oxide content and 4.0 micron average size.

### Example 11

A mixture containing 180 ml of deionized water, 2 ml of styrene and 20 ml of 8.6 % w/v magnetic metal oxide, prepared as described in Example 1, was placed in a sealed bottle. The bottle was evacuated and rotated at about 60 rpm in a 55 °C oven for one hour. To the mixture were added 4 g of potassium persulfate and 160 ml of 6.8 % w/v magnetic particles (3.0 micron, 14 % metal oxide content), prepared as described in Example 10. The bottle was resealed, evacuated and rotated for one hour and added 10 ml of 2 % sodium dodecylsulfate. After 5 more hours 2 ml of styrene and 2 g of potassium persulfate were added to the mixture. The mixture was rotated for another fifteen hours, filtered through two layers of cheese cloth, separated magnetically and washed several times with deionized water until the supernatant was clear. The resulting magnetic particles were resuspended to 160 ml with deionized water to give a 7.78 % w/v suspension with about 19 % metal oxide content and 4.2 micron average size.

### Example 12

A mixture containing 90 ml of deionized water, 1 ml of styrene and 10 ml of 8.6 % w/v magnetic metal oxide, prepared as described in Example 1, was placed in a sealed bottle. The bottle was evacuated and rotated at about 60 rpm in a 55 °C oven for one hour. To the mixture were added 1 g of potassium persulfate and 80 ml of 7.78 % w/v magnetic particles (3.2 micron, 19 % metal oxide content), prepared as described in Example 11. The bottle was resealed, evacuated and rotated for four hour and added 5 ml of 2 % sodium dodecylsulfate. After 5 more hours 1 ml of styrene and 1 g of potassium persulfate were added to the mixture. The mixture was rotated for another fifteen hours, filtered through two layers of cheese cloth, separated magnetically and washed several times with deionized water until the supernatant was clear. The resulting magnetic particles were resuspended to 160 ml with deionized water to give a 4.5 % w/v suspension with about 23 % metal oxide content and 4.5 micron average size.

### Example 13

A mixture containing 400 ml of deionized water, 1.92 ml of styrene, 0.08 ml of divinyl benzene, 4 g of potassium persulfate, 20 g of 200 - 400 mesh 4 % divinyl benzene cross linked polystyrene beads and 10 ml of 8.6 % w/v magnetic metal oxide, preapred as described in Example 1, was placed in a sealed bottle. The bottle was evacuated and rotated at about 60 rpm in a 55 °C oven for 15 hours. The mixture was allowed to settle and the supernatant was decanted. The resulting magnetic beads were resuspended in 200 ml of deionized water and allowed to settle again. This process was repeated several times until the supernatant was clear. The resulting magnetic beads were resuspended in 200 ml of deionized water and added 0.1 g of sodium dodecyl sulfate, 2.0 g of potassium persulfate, 0.48 ml of styrene, and 0.02 ml of divinyl benzene. The bottle was resealed, evacuated and rotated at about 60 rpm in a 55 °C over for one hour and added a solution containing 0.098 ml of undecylenic acid and 0.002 ml of divinyl benzene in 0.4 ml of methanol. The mixture was rotated for four more hours and cleaned up by gravitational sedimentation as described previously. The water was removed by filtration and the carboxyl magnetic beads were dried to give 20 g of 200 - 400 mesh carboxyl magnetic beads.

### Example 14

A mixture containing 100 ml of deionized water, 0.5 ml of styrene, 2 g of potassium persulfate, 75 ml of 5 % w/v 4.0 micron polystrene particles and 10 ml of 6.88 % w/v magnetic metal oxide, prepared as descrived in Example 4, was placed in a sealed bottle. The bottle was evacuated and rotated at about 60 rpm in a 55 °C oven for fifteen hours. The mixture was filtered through two layers of cheese cloth, separated magnetically and washed several times with deionized water until the supernatant was clear. The resulting magnetic particles were resuspended to 150 ml with deionized to give a 2.5 % w/v suspension with about 14 % metal oxide content and 4.3 micron average size.

### Example 15

Same procedures as described in Example 14 were followed except 20 ml of 6.88 % w/v magnetic metal oxide, prepared as described in Example 4, was used to give 160 ml of 2.5 % w/v suspension with about 18 % metal oxide content and 4.3 micron average size.

### Example 16

A mixture containing 2000 ml of deionized water, 13 ml of styrene and 550 ml of 2.98 % w/v magnetic metal oxide prepared as described in Example 3, was placed in a sealed bottle. The bottle was evacuated and rotated at about 60 rpm in a 55 °C oven for one hour. To the mixture were added 20 g of potassium persulfate and 950 ml of 10 % w/v, 3.0 micron polystyrene particles. The bottle was resealed, evacuated and rotated for one hour and added 60 ml of 2 % sodium dodecylsulfate. After five more hours 8 ml of styrene and 10 g of potassium persulfate were added to the mixture. The mixture was rotated for another fifteen hours, filtered through two layers of cheese cloth, separated magnetically and washed several times with deionized water until the supernatant was clear. The resulting magnetic particles were resuspended to 3000 ml with deionized water to give a 3.38% w/v suspension with about 12 % magnetic metal oxide content and 3.2 micron average size.

### Example 17

A mixture containing 150 ml of magnetic particles (3.2 micron, 3.38 % w/v with 12 % metal oxide content) prepared as described in Example 16, 2 ml of 1 % NP 40, 0.5 ml of methyl methacrylate or styrene, 1 g of potassium persulfate and 2 ml of functionalized monomer, trimethylammoniumethyl methacrylate methosulfate (40 % aqueous solution), was placed in a sealed bottle. The bottle was rotated at about 60 rpm in a 55 °C oven for four hours. The mixture was filtered through two layers of cheese cloth, separated magnetically and washed several times with deionized water until the supernatant was clear. The resulting magnetic particles were resuspended to 200 ml with deionized water to give a 2.5 % w/v suspension of magnetic particles with trimethylammonium functional groups on the surface.

### Example 18

Same procedures as described in Example 17 were followed except 1 ml of functionalized monomer, 2-aminoethyl methacrylate, was used to give 200 ml of 2.5 % w/v suspension of magnetic particles with amino groups on the surface.

### Example 19

Same procedures as described in Example 17 were followed except 1 ml of functionalized monomer, 2-hydroxyethyl methacrylate, was used to give 200 ml of 2.5 % w/v suspension of magnetic particles with hydroxyl groups on the surface.

### Example 20

Same procedures as described in Example 17 were followed except 1 ml of monomer, 1-vinyl-2-pyrrolidinone, was used to give 200 ml of 2.5 % w/v suspension of magnetic particles with polyvinylpyrrolidinone on the surface.

### Example 21

Same procedures as described in Example 17 were followed except 1 g of functionalized monomer, methyl propene sulfonic acid, was used to give 200 ml of 2.5 % w/v suspension of magnetic particles with sulfonic acids groups on the surface.

### Example 22

Same procedures as described in Example 17 were followed except 1 ml of functionalized monomer, dimethylaminoethyl methacrylate, was used to give 200 ml of 2.5 % w/v suspension of magnetic particles with dimethylamino groups on the surface.

### Example 23

A mixture containing 20 ml of 7.0 % w/v, 2.11 micron polystyrene particles, 100 ml of 1.8 % w/v metal oxide prepared as described in Example 5, 50 ml of deionized water and a solution containg 0.15 g of benzoyl peroxide in 7.5 ml of styrene was placed in a sealed bottle. The bottle was evacuated and rotated at about 60 rpm in a 55 °C oven for fifteen hours. The mixture was filtered through two layers of cheese cloth, separated magnetically and washed several times with deionized water until the supernatant was clear. The resulting magnetic particles were resuspended to 200 ml with deionized water to give 5.0 % w/v suspension with about 16.8 % metal oxide content and 3.6 micron average size.

### Example 24

A mixture containing 20 ml of 7.0 % w/v, 2.11 micron polystyrene particles, 100 ml of 1.8 % w/v metal oxide prepared as described in Example 5, 50 ml of deionized water and a solution containing 0.15 g of benzoyl peroixde and 0.75 ml of divinyl benzene in 6.75 ml of styrene was placed in a sealed bottle. The bottle was evacuated and rotated at about 60 rpm in a 55 °C oven for fifteen hours. The mixture was filtered throught wo layers of cheese cloth, separated magnetically and washed several times with deionized water until the supernatant was clear. The resulting crosslinked magnetic particles were resuspended to 200 ml with deionized water to give 5.0 % w/v suspension with about 16.8 % metal oxide content and 3.6 micron average size. The crosslinked magnetic particles prepared this way were found to be uniform in size and inert to common organic solvents such as acetone, acetonitrile and dimethyl formamide.

### Example 25

A mixture containing 20 ml of 7.0 % w/v, 2.11 micron polystyrene particles, 150 ml of 1.8 % w/v metal oxide prepared as described in Example 5 and a solution containing 0.15 g of benzoyl peroxide, 0.75 ml of divinyl benzene in 6.75 ml of styrene was placed in a sealed bottle. The bottle was evacuated and rotated at about 60 rpm in a 55 °C oven for fifteen hours. The mixture was filtered through two layers of cheese cloth, separated magnetically and washed several times with deionized water until the supernatant was clear. The resulting crosslinked magnetic particles were resuspended to 200 ml with deionized water to give 5.4 % w/v suspension with about 23 % metal oxide content and 4.0 micron average size. The crosslinked magnetic particles prepared this way were found to be uniform in size and inert to common organic solvents such as acetone, actonitrile and dimethyl formamide.

### Example 26

A mixture containing 15 ml of 9.16 % w/v, 3.2 micron polystyrene particles, 100 ml of 1.8 % w/v metal oxide prepared as described in Example 5, 55 ml of deionized water and a solution containing 0.15 g of benzoyl peroxide and 0.75 ml of divinyl benzene in 6.75 ml of styrene was placed in a sealed bottle. The bottle was evacuated and rotated at about 60 rpm in a 55 °C oven for fifteen hours. The mixture was filtered through two layers of cheese cloth, separated magnetically and washed several times with deionized water until the supernatant was clear. The resulting crosslinked magnetic particles were resuspended to 200 ml with deionized water to give 4.7 % w/v suspension with about 16.8 % metal oxide content and 5.5 micron average size., The crosslinked magnetic particles prepared this way were found to be uniform in size and inert to common organic solvents such as acetone, actonitrile and dimethyl formamide.

### Example 27

A mixture containg 30 ml of 4.5 % w/v, 4.1 micron polystyrene particles, 100 ml of 1.8 % w/v metal oxide prepared as described in Example 5, 40 ml of deionized water and a solution containing 0.15 g of benzoyl peroxide and 0.75 ml of divinyl benzene in 6.75 ml of styrene was placed in a sealed bottle. The bottle was evacuated and rotated at about 60 rpm in a 55 °C oven for fifteen hours. The mixture was filtered through two layers of cheese cloth, separated magnetically and washed several times with deionized water until the supernatant was clear. The resulting crosslinked magnetic particles were resuspended to 200 ml with deionized water to give 4.5 % w/v suspension with about 16.9 % metal oxide content and 6.7 micron average size. The crosslinked magnetic particles prepared this way were found to be unform in size and inert to common organic solvents such as acetone, acetonitrile and dimethyl formamide.

### Example 28

A mixture containing 20 ml of 7.0 % w/v, 2.11 micron polystyrene particles, 100 ml of 1.8 % w/v metal oxide prepared as described in Example 5, 50 ml of deionized water and a solution containing 0.15 g of benzoyl peroixde, 0.75 ml of undecylenyl alcohol and 0.75 ml of divinyl benzene in 6 ml of styrene was placed in a sealed bottle. The bottle was evacuated and rotated at about 60 rpm in a 55 °C oven for fifteen hours. The mixture was filtered through two layers of cheese cloth, separated magnetically and washed several times with deionized water until the supernatant was clear. The resulting crosslinked hydroxyl magnetic particles were filtered and dried to give 9 g of powder with about 16.8 % metal oxide content and 3.9 micron average size. The crosslinked hydroxyl magnetic particles prepared this way were found to be uniform in size and inert to common organic solvents such as acetone, acetonitrile and dimethyl formamide.

### Coupling Biological Materials to Magnetic Particle

### Example 29

In a 80 ml bottle was placed 30 ml of 4.3 micron, 5.0 % w/v carboxyl magnetic particles prepared as described in Example 7. The particles were separated magnetically and resuspended in 50 ml of phosphate buffer (0.1 M. pH 5.5). To the particle suspension were added 20 mg of bovine serum albumin and 100 mg of 1-ethyl-3-(3-dimethylaminopropyl)-carbodiimide (EDC). The mixture was rotated end to end at room temperature for two hours and separated magnetically. The particles were washed once with 80 ml of phosphate buffer and resuspended to 75 ml with phosphate buffered saline (0.1 M, pH 7.0) to give a 2.0 % w/v suspension.

To couple bovine serum albumin to magnetic particles by passive adsorption the same procedures were followed except no EDC was used.

### Example 30

In a 4 ml vial was placed 1 ml of 4.3 micron, 5.0 % w/v carboxyl magnetic particles prepared as described in Example 7. The particles were separated magnetically and washed once with 2 ml of phosphate buffer (0.1 M, pH 5.5) and resuspended to 2 ml with the same buffer. To the particles suspension were added 140 ml of 1.4 mg/ml Goat (Gt) anti Mouse (Ms) IgG and 10 mg of 1-ethyl-3-(3-dimethylaminopropyl) carbodiimide. The vial was rotated end to end at room temperature for two hours. The particles were separated magnetically, washed once with 2 ml of phosphate buffer and resuspended to 2 ml with phosphate buffered saline (0.1 M, pH 7.0) to give a 2.5 % w/v Gt anti Ms IgG coated magnetic particles. Other kind of antibody either monoclonal or polyclonal could also be coupled to carboxyl magnetic particles by using the same procedures.

To couple Gt anti Ms IgG or other kind of antibody to the magnetic particles by passive adsorption the same procedures were followed except no EDC was used.

### Example 31

In a 4 ml vial was placed a 2.5 ml of bovine serum albumin coated magnetic particles (4.3 micron, 2 % w/v) prepared as described in Example 29. The particles were separated magnetically and resuspended to 2 ml with phosphate buffer (0.1 M, pH 5.5). To the mixture were added 10 ul of Ms anti B red cells surface antigen (20 mg/ml) and 1 mg of 1-ethyl-3-(3-dimethylaminopropyl)-carbodiimide. The mixture was rotated end to end at room temperature for two hours. The particles were separated magnetically, washed once with phosphate buffer and resuspended in 2 ml of phosphate buffered saline (0.1 M, pH 7.0) to give a 2.5 % w/v suspension.

### Example 32

Same procedures as described in Example 31 were followed except using 40 ul of Ms anti A red cells surface antigen (5 mg/ml) to give 2 ml of 2.5 % w/v suspension.

### Blood Typing Using Magnetic Particles

### Example 33

In a 5mm x 65mm test tube labeled A was placed 25 ul of 2.5 % w/v Ms anti A coated magnetic particles prepared as described in Example 32. To the other test tube labeled B was placed 25 ul of 2.5 % w/v Ms anti B coated magnetic particles prepared as described in Example 31. To both test tubes was added 50 ul of 1 % packed red blood cells prepared by 1 to 100 dilution of packed red blood cells in isotonic buffered saline. The test tubes were shaked by finger tapping for several times and placed on the top of a magnet. The results were summarized as follows:

| | BLOOD TYPE | | | |
|---|---|---|---|---|
| | A | B | O | AB |
| TUBE A | + | - | - | + |
| TUBE B | - | + | - | + |

Where + represent a positive reaction, meaning the red cells were agglutinated by the corresponding antibody coated magnetic particles as a result the supernatant in the test tube was clear after magnetic separation. On the other hand the supernatant of a negative reaction would remain cloudy after magnetic separation due to the absence of agglutination between the red cells and the antibody coated magnetic particles.

### Immunoassays Using Magnetic Particles

### Example 34

In a 2 ml microcentrifuge tube was placed 1 ml of 6 % w/v, 3 micron carboxyl magnetic particles. The particles were centrifuged for 3 minutes at 10000 rpm. The supernatant was aspirated and the particles were resuspended by vortexing with 1 ml of 5 to 100 ug/ml recombinant HBcAg in acetate buffer. The tube was rotated at toom temperature for two hours and centrifuged as described before. The supernatant was aspirated and the particles were resuspended in 1 ml of overcoat solution containing acetate buffer and 2 to 10 % of normal animal serum. The tube was rotated at room temperature for 2 to 16 hours and centrifuged as described before. The supernant was aspirated and the particles were washed three times with 1 ml of isotonic buffered slaine (IBS) by centrifugation and resuspension. Finally, the particles were resuspended with 1 ml of IBS and stored at 2 to 8°C.

### Example 35

To the first two columns of a 96-well microtiter plate was placed 20 ul of 0.25% w/v heptitis B core antigen (HBcAg) coated magnetic particles prepared as described in Example 34. Sample preparation consisted of various dilutions of a HBcAb positive serum into a negative plasma, followed by a 1:100 dilution of each sample into specimen dilution buffer (SDB). The SDB contained phosphate buffer, protein stabilizers, detergentand antimicrobial agents. To the wells containing the particles were added 50 ul of each final sample dilution. After 30 minutes incubation at 37°C, the particles were separated for two minutes on a magnetic separator and washed three times with 200 ul wash buffer containing salts and detergent. To each well containing the particles was added 50 ul of goat antihuman IgG-B-D-galactosidase conjugate (0.5 ug/ml) in diluent containing salts, protein stabilizers, glycerol, detergent and antimicrobial agents. After 15 minutes incubation at 37°C the particles were separated and washed three times as described above and resuspended in 30 ul of IBS. The particles were transferred to the first two columns of a black microtiter plate (Dynatech). To each well containing the particles was added 100 ul of a solution containing 4-methylumbelliferyl-B-galactopyranoside (MUG, Sigma). The plate was incubated at 37°C and the fluorescence intensity was mesured by using a Fluorescence Concentration Analyzer (FCA, Pandex) equipped with 365 nm excitation and 450 nm emmision filters at five minutes interval and 10 X gain setting. The increase in fluorescence intentsity in a five minutes interval was recorded in arbitrary fluorescence unit (AFU) and presented in Table I.

**TABLE I**

| Dilution of Positive Specimen | AFU (5 Minutes) Average of Two Wells |
|---|---|
| 1:100 | 22687 |
| 1:1000 | 5933 |
| 1:5000 | 1516 |
| 1:8000 | 835 |
| 1:10000 | 639 |
| 1:15000 | 495 |
| 1:20000 | 427 |
| 1:25000 | 307 |

### Example 36

The coupling of mouse antiHBsAg to carboxyl magnetic particles was similar to Example 30.

To the wells of a black 96-well microtiter plate (Dynatech) were added 20 ul of 0.25% w/v, 3.2 micron, mouse antiHBsAg coated carboxyl magnetic particles in duplicate. To the wells containing the magnetic particles was added 100 ul of neat plasma containing various amounts of HBsAg or a HBsAg-negative plasma. After 30 minutes incubation at 37°C, the particles were separated for two minutes on a magentic separator and washed once with 100 ul of wash buffer containing salts and detergent. To each will containing the particles was added 20 ul of mouse antiHBsAg--B-galactosidase conjugate in diluent containing salts, protein stabilizers, glycerol, detergent and antimicrobial agents. After 15 minutes incubation at 37°C, the particles were separated and washed five times as described above. To each will containing the particles was added 50 ul of a solution containing 4-methylumbelliferyl-B-D-galactopyranoside (MUG, Sigma). The plate was incubated at 37°C and the fluorescence intentisty was measured by using a Fluorescence Concentration Analyzer (FCA, Pandex) equipped with 365 nm excitation and 450 nm emission filters ar five minutes interval and 10 X gain setting. The increase in fluorescence intentsity in a five minutes interval was recorded in arbitrary fluorescence unit (AFU) and presented in Table II.

**TABLE II**

| HBsAg Conc. (nano gm) | AFU (5 Minutes) Average of Two Wells |
|---|---|
| 1.0 | 1149 |
| 0.5 | 455 |
| 0.25 | 218 |
| 0.125 | 118 |
| neg. | 14 |

### Example 37

The HIV-1 antigens from HTLV-IIIB/H-9 cells (Gallo Strain) were coupled to 3.6 micron carboxyl magnetic particles by using similar procedures as described in Example 34.

To the wells of a 96-well microtiter plate were added 20 ul of 0.25% w/v of HIV coated magnetic particles in duplicate. To the wells containing the particles were added 50 ul of positive, borderline and negative specimens diluted 1:100 in specimen dilution buffer (SDB) containing phosphate buffer, protein stabilizers, detergent and antimicrobial agents. After 30 minutes incubation at 37°C, the particles were separated for two minutes on a magnetic separator and washed three times with 100 ul of washed buffer containing salts and detergent. To each well containing particles was added 50 ul of goat antihuman-B-galactosidase (approximately 0.5 ug/ml) conjugate in diluent containing salts, protein stabilizers, glycerol, detergent and antimicrobial agents. After 15 minutes incubation at 37°C, the particles were washed four times as described above. The particles were transferred to the black microtiter plate (Dynatech). To each well containing particles was added 100 ul of a solution containing 4-methylumbelliferyl-B-D-galactopyranoside (MUG, Sigma). The plate was incubated at 37°C and the fluorescence intensity was measured by using a Fluorescence Concentration Analyzer (FCA, Pandex) equipped with 365 nm excitation and 450 nm emission filters at five minutes intervals and 25 X gain setting. The increase in fluorescence intensity in a five minutes interval was recorded in arbitrary fluorescence unit (AFU) and presented in Table III.

**TABLE III**

| Anti-HIV Specimens | AFU (5 minutes) Average of Two Wells |
|---|---|
| Positive Control | 9462 |
| Borderline Specimen | 527 |
| Negative Control | 86 |

### Cell Separation Using Magnetic Particles

### Example 38

The 4.3 micron carboxyl magnetic particles prepared as described in Example 7 were washed and sonicated in phosphate buffered saline (PBS, pH 7.7), sterilized in 70% ethanol for 10 minutes, washed three times in PBS and incubated for 48 hours at 4°C with affinity-purified sheep anti-mouse immunoglobulin antibody (SAM) at 0.5 mg/ml and a ratio of 3.3 mg antibody/100mg particles. Before use, the antibody coated magnetic particles were washed in PBS and resuspend at the desired concentration in PBS.

Human tissue culture cALLa-positive NALM-16 leukemia cells were washed and suspended in PBS. One fraction was not treated with antibody (-MoAb). The other fraction was treated with two anti-CD10 and one anti-CD9 monoclonal antibodies (+MoAb) for 30 minutes at 4°C, washed in PBS and adjusted to 3.5 x 10⁶ cells/ml on PBS. To two tubes, one containing the antibody treated cells (+MoAb), the other containing untreated cells (-MoAb) were added SAM coated magnetic particles at a particle to starting cell ratio of 45. The tubes were rotated at 4°C for 30 minutes. The particles were separated with a magnetic separator. The supernatant was collected and centrifuged to collect the remaining cells. The pellet was resuspended in 100 ul of trypan blue and total cell count was made. The results were presented in Table IV.

**TABLE IV**

| Particle/cell Ratio | Cells +/-MoAb | Cells Received | % Depletion |
|---|---|---|---|
| 0 | + | 7.62x10⁵ | 0 (Control) |
| 45 | + | 2.89x10⁴ | 96.2 |
| 45 | - | 7.33x10⁵ | 4.6 |

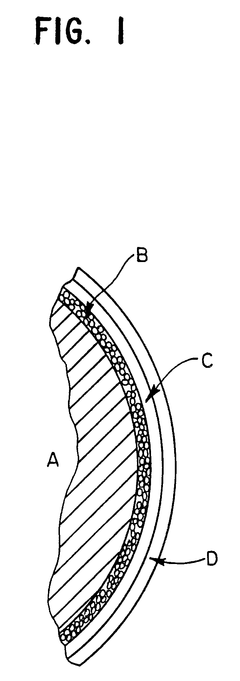

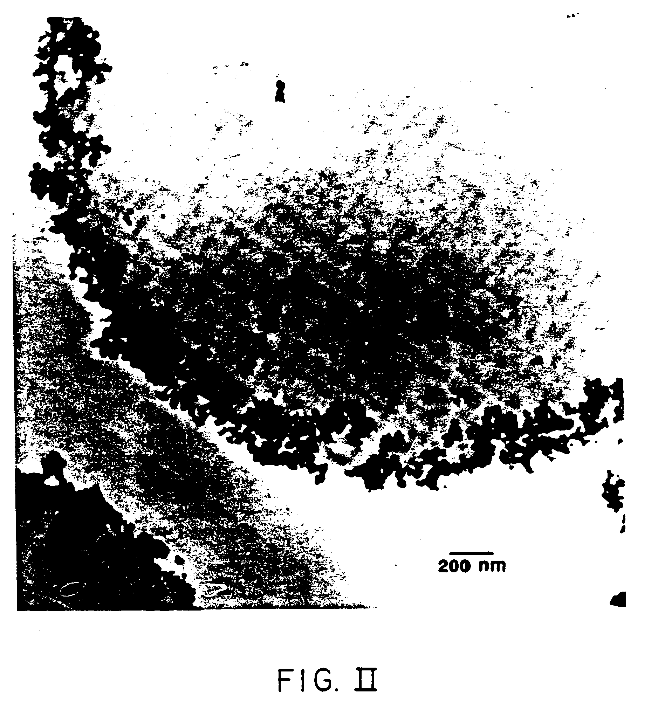

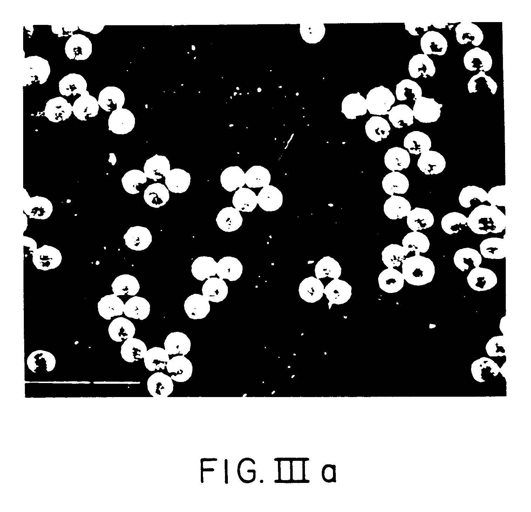

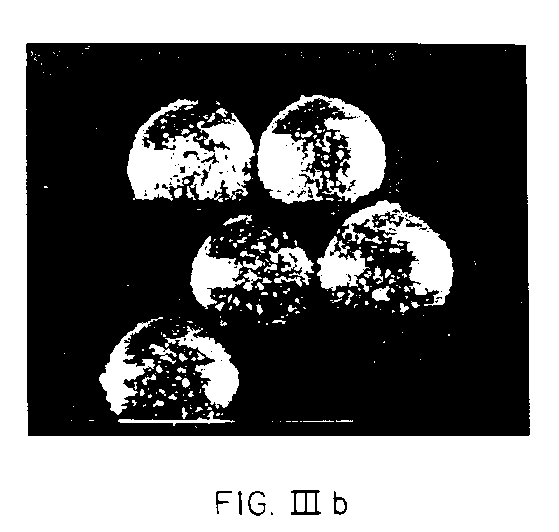

## Claims

1. Monodispersed magnetic particles of a uniform size distribution and magnetic content comprising:
an inner core polymer particle able to adsorb a monomer; and
a magnetically responsive metal oxide and polymer combination prepared by polymerisation of a mixture of metal oxide and monomer in the presence of initiator in the mixture, said polymer being comprised of monomers able to adsorb to said inner core polymer particle, said metal oxide and polymer combination evenly covering said inner core particle, said magnetic particles being of a uniform size distribution, uniform magnetic content, and monodispersed in solution.

2. The magnetic particles of Claim 1, including an outer polymer coating covering said magnetically responsive metal oxide and polymer combination.

3. The magnetic particles of Claim 2, including a layer of functionalized polymer covering said outer polymer coating.

4. The magnetic particle of Claim 2 or 3 wherein said outer polymer coating comprises a polymer selected from the group consisting of polystyrene, cross-linked polystyrene or functionalized polystyrene.

5. The magnetic particles of Claim 1, including a layer of functionalized polymer covering said metal oxide and polymer combination, said magnetic particles being of a uniform size distribution, uniform magnetic content and monodispersed in solution.

6. The magnetic particles of Claim 3, 4 (when dependant on Claim 3) or 5 wherein said functionalized polymer is a compound which provides carboxyl, amino or hydroxyl functional groups for coupling to biological material.

7. The magnetic particles of Claim 3, 4 (when dependant on Claim 3) or 5 wherein said functionalized polymer is coupled to biological material.

8. The particle of any one of the above claims wherein said core particle comprises polystyrene or cross-linked polystyrene.

9. The particle of any one of the above claims wherein said core particle ranges from about 1 to 100 microns.

10. The particle of any one of the preceding claims having at least one additional metal oxide and polymer combination coating evenly covering said first metal oxide and polymer combination coating.

11. The particle of any one of the above claims wherein said magnetically responsive metal oxide of at least one said coating comprises particles of diameter about 1 micron or less.

12. The particle of any one of the above claims wherein at least one said magnetically responsive metal oxide comprises either superparamagnetic, paramagnetic, or ferromagnetic metal oxide.

13. The particle of any one of the above claims wherein at least one said responsive metal oxide and polymer combination comprises polystyrene, cross-linked polystyrene, functionalized polymer.

14. The particle of any one of Claims 1 to 4 wherein the metal oxide of at least one said coating is formed from transition metal salts.

15. A process of making a magnetically responsive polymer particle having a polymeric core particle evenly covered by a metal oxide and polymer combination coating comprising:
collecting metal oxide particles of diameter about 1 micron or less;
mixing said metal oxide particles with a monomer to form metal oxide and polymer combination coating; and
coating a polymeric core particle in the presence of an initiator with said metal oxide and polymer combination coating.

16. The process of Claim 15 wherein said metal oxide is produced by:
(a) heating and precipitating a mixture of divalent and trivalent transition metal salts with sodium hydroxide;
(b) washing precipitate metal oxide until neutral in pH;
(c) resuspending said metal oxide in deionized water;
(d) stirring said metal oxide to break down the aggregate of metal oxide crystals; and
(e) low speed centrifugation to narrow the size of said metal oxide crystals.

17. The process of any one of Claims 15 or 16 wherein the metal oxide and polymer combination particle is mixed with a monomer or monomers to form a metal oxide and polymer combination coating, said monomer being selected from the group consisting of styrene, divinylbenzene, methyl methacrylate, glycidyl methacrylate, and other olefinic monomers.

18. The process of any one of Claims 15 to 17 wherein the polymeric core particle is between 1 to 100 microns.

19. A process for the determination of presence or concentration of an analyte comprising:
contacting magnetic particles of Claim 3 or 5;
having a ligand specific for said analyte attached to said functionalized polymer with fluid specimen to form a suspension;
incubating said suspension until sufficient analyte has reacted with said specific ligand; separating said magnetic particles from said suspension;
adding a second labelled ligand specific for said analyte to said separated magnetic particles;
incubating said suspension until sufficient analyte has reacted with said second labelled ligand specific for said analyte;
separating said magnetic particles from said suspension;
measuring the amount of labelled ligand associated with said magnetic particles;
relating the amount of labelled ligand measured with the amount of analyte measured for a control sample.

20. The process of Claim 19 wherein second ligand is labelled with β-D-galactosidase and the amount of labelled ligand associated with said magnetic particle is measured using the substrate 4-methylumbelliferyl-β-galactopyranoside and a fluorescence analyzer.

21. The process of Claim 20 wherein the fluorescence analyzer has about 365 nm excitation and about 450 nm emission filters.

22. The process of any one of Claims 19 to 21 wherein the analyte is selected from the group consisting of enzymes, hormones, peptides, vitamins, nucleic acids, oligonucleotides, biological cells, antigens, antibodies and haptens.

23. The process of any one of Claims 19 to 22 wherein the particle comprises bovine serum albumin attached to said functionalized polymer.

24. A process to determine the presence or concentration of specific nucleic acid sequences in nucleic acid target molecules comprising:
contacting magnetic particles of Claim 3 or 5 having a nucleic acid complementary to said nucleic acid sequence of said target molecule, attached to said functionalized polymer, with a fluid specimen to form a suspension;
incubating said suspension under hybridizing conditions for a period of time sufficient to permit hybridization;
separating said magnetic particles from said suspension;
adding a second labelled nucleic acid sequence complementary to said nucleic acid sequence of said target molecule;
incubating said suspension under hybridizing conditions for a period of time sufficient to permit hybridization;
separating said magnetic particles from said suspension; and
detecting duplex formation on said magnetic particles by means of said label.

25. The process of Claim 24 wherein said labelled nucleic acid sequence complementary to said nucleic acid sequence of said target molecule is labelled with biotin and said label is amplified by a labelled avidin.

26. The process of Claim 24 wherein second labelled nucleic acid sequence is labelled with β-D-galactosidase and the amount of labelled nucleic acid sequence associated with said magnetic particle is measured using the substrate 4-methylumbelliferyl-β-galactopyranoside and a fluorescence analyzer.

27. The process of Claim 26 wherein the fluorescence analyzer has about 365 nm excitation and about 450 nm emission filters.

28. A process for isolating a biosubstance comprising:
contacting magnetic particles of Claim 3 or 5 having a ligand specific for said biosubstance;
incubating said suspension until sufficient biosubstance has reacted with said ligand;
separating said magnetic particles from said suspension;
separating said magnetic particles from said biosubstance; and
obtaining substantially pure biosubstance.

29. A process for removing unwanted biosubstance comprising:
contacting magnetic particles of Claim 3 or 5 having a ligand specific for said biosubstance;
incubating said suspension until sufficient biosubstance has reacted with said ligand;
separating said magnetic particles from said suspension; and
obtaining suspension free of unwanted biosubstance.

30. The process of Claim 28 or 29 wherein said biosubstance is a protein.

31. The process of Claim 28 or 29 wherein said biosubstances are biological cells.

32. The process of Claim 31 wherein said biosubstance comprises bone marrow cells.

33. A process for industrial waste purification comprising removing unwanted substances from industrial waste using the magnetic particles of Claim 3 or 5.

## Patentansprüche

1. Monodisperse magnetische Teilchen mit einheitlicher Größenverteilung und magnetischen Gehalt, umfassend:
ein Innenkernpolymerteilchen, das zur Adsorbtion eines Monomers in der Lage ist; und
eine Kombination aus magnetisch reagierendem Metalloxid und Polymer, hergestellt durch Polymerisation einer Mischung aus Metalloxid und Monomer in Gegenwart eines in der Mischung vorliegenden Initiators, wobei das Polymer aus Monomeren besteht, die in der Lage sind, am Innenkernpolymerteilchen zu adsorbieren, wobei die Kombination aus Metalloxid und Polymer das Innenkernteilchen gleichmäßig bedeckt und die magnetischen Teilchen eine einheitliche Teilchengrößenverteilung aufweisen, einen einheitlichen magnetischen Gehalt besitzen und in Lösung monodispergiert sind.

2. Magnetische Teilchen nach Anspruch 1,
die einen äußeren Polymerüberzug aufweisen, welcher die Kombination aus magnetisch reagierendem Metalloxid und Polymer überdeckt.

3. Magnetische Teilchen nach Anspruch 2,
die eine den äußeren Polymerüberzug bedeckende Schicht aus funktionalisiertem Polymer aufweisen.

4. Magnetische Teilchen nach Anspruch 2 oder 3,
wobei der äußere Polymerüberzug ein Polymer aufweist, das aus der Gruppe ausgewählt ist, die aus Polystyrol, vernetztem Polystyrol oder funktionalisiertem Polystyrol besteht.

5. Magnetische Teilchen nach Anspruch 1,
die eine die Kombination aus Metalloxid und Polymer bedeckende Schicht aus funktionalisiertem Polymer aufweisen, wobei die magnetischen Teilchen eine einheitliche Größenverteilung aufweisen, einen einheitlichen magnetischen Gehalt besitzen und in Lösung monodispergiert sind.

6. Magnetische Teilchen nach Anspruch 3, 4 (wenn abhängig von Anspruch 3) oder 5,
wobei das funktionalisierte Polymer eine Verbindung ist, welche funktionelle Carboxyl-, Amino- oder Hydroxylgruppen für die Kupplung mit biologischem Material bereitstellt.

7. Magnetische Teilchen nach Anspruch 3, 4 (wenn abhängig von Anspruch 3) oder 5,
wobei das funktionalisierte Polymer mit biologischem Material gekoppelt ist.

8. Teilchen nach mindestens einem der vorhergehenden Ansprüche,
wobei das Kernteilchen Polystyrol oder vernetztes Polystyrol umfaßt.

9. Teilchen nach mindestens einem der vorhergehenden Ansprüche,
wobei das Kernteilchen im Bereich von etwa 1 bis 100 µm liegt.

10. Teilchen nach mindestens einem der vorhergehenden Ansprüche mit mindestens einem zusätzlichen Überzug aus einer Kombination aus Metalloxid und Polymer, der den ersten Überzug aus einer Kombination aus Metalloxid und Polymer bedeckt.

11. Teilchen nach mindestens einem der vorhergehenden Ansprüche,
wobei das magnetisch reagierende Metalloxid von mindestens einem der Überzüge Teilchen mit einem Durchmesser von etwa 1 µm oder weniger aufweist.

12. Teilchen nach mindestens einem der vorhergehenden Ansprüche,
wobei das magnetisch reagierende Metalloxid entweder superparamagnetisches, paramagnetisches oder ferromagnetisches Metalloxid aufweist.

13. Teilchen nach mindestens einem der vorhergehenden Ansprüche,
wobei mindestens eine Kombination aus magnetisch reagierenden Metalloxid und Polymer Polystyrol, vernetztes Polystyrol, bzw. funktionalisiertes Polymer aufweist.

14. Teilchen nach mindestens einem der Ansprüche 1 bis 4, wobei das Metalloxid von mindestens einem der Überzüge aus Übergangsmetallsalzen gebildet ist.

15. Verfahren zur Herstellung von magnetisch reagierenden Polymerteilchen mit einem polymeren Kernteilchen, das gleichmäßig mit einem Überzug aus einer Kombination aus Metalloxid und Polymer bedeckt ist, das folgende Schritte aufweist:
- Sammeln von Metalloxidteilchen mit einem Durchmesser von etwa 1 µm oder weniger;
- Mischen der Metalloxidteilchen mit einem Monomer zur Bildung eines Überzugs aus der Kombination aus Metalloxid und Polymer; und
- Beschichten eines polymeren Kernteilchens in Gegenwart eines Initiators mit dem Überzug aus der Kombination aus Metalloxid und Polymer.

16. Verfahren nach Anspruch 15,
wobei das Metalloxid durch folgende Maßnahmen gebildet wird:
(a) Erwärmen und Niederschlagen einer Mischung von zweiwertigen und dreiwertigen Übergangsmetallsalzen mit Natriumhydroxid;
(b) Waschen des Metalloxidniederschlags, bis er einen neutralen pH-Wert aufweist;
(c) Erneutes Suspendieren des Metalloxids in deionisiertem Wasser;
(d) Rühren des Metalloxids, um das Aggregat aus Metalloxidkristallen zu zerbrechen; und
(e) Zentrifugieren bei niedriger Geschwindigkeit, um die Größe der Metalloxidkristalle einzuengen.

17. Verfahren nach mindestens einem der Ansprüche 15 oder 16, wobei das Teilchen aus der Kombination aus Metalloxid und Polymer mit einem Monomer oder mehreren Monomeren vermischt wird, um einen Überzug aus der Kombination aus Metalloxid und Polymer zu bilden, wobei das Monomer aus der Gruppe ausgewählt wird, die aus Styrol, Divinylbenzol, Methylmethacrylat, Glycidylmethacrylat und anderen olefinischen Monomeren besteht.

18. Verfahren nach mindestens einem der Ansprüche 15 bis 17, wobei das polymere Kernteilchen im Bereich von 1 bis 100 µm liegt.

19. Verfahren zur Bestimmung der Anwesenheit oder Konzentration eines Analyts, das folgende Schritte aufweist:
Kontaktieren der magnetischen Teilchen gemäß Anspruch 3 oder 5,
die einen am funktionalisierten Polymer gebundenen Liganden tragen, mit einer Fluidprobe zur Bildung einer Suspension;
Inkubieren der Suspension bis ausreichend Analyt mit dem spezifischen Liganden reagiert hat;
Abtrennen der magnetischen Teilchen von der Suspension;
Hinzugeben eines zweiten markierten Liganden, der für den Analyten spezifisch ist, zu den abgetrennten magnetischen Teilchen;
Inkubieren der Suspension bis ausreichend Analyt mit dem zweiten markierten Liganden, der für den Analyten spezifisch ist, reagiert hat;
Abtrennen der magnetischen Teilchen von der Suspension;
Bestimmen der Menge des markierten Liganden, der an den magnetischen Teilchen gebunden ist;
Inbeziehungsetzen der gemessenen Menge an markiertem Liganden zur gemessenen Menge an Analyt bei einer Kontrollprobe.

20. Verfahren nach Anspruch 19,
wobei der zweite Ligand mit β-D-Galactosidase markiert wird und die Menge des an dem magnetischen Teilchen gebundenen, markierten Liganden unter Verwendung des Substrates 4-Methylumbelliferyl-β-galactopyranosid und eines Fluoreszenz-Analysegerätes bestimmt wird.

21. Verfahren nach Anspruch 20,
wobei das Fluoreszenz-Analysegerät ein Erregungsfilter von etwa 365 nm und ein Emissionfilter von etwa 450 nm besitzt.

22. Verfahren nach einem der Ansprüche 19 bis 21,
wobei der Analyt aus der Gruppe ausgewählt wird, die aus Enzymen, Hormonen, Peptiden, Vitaminen, Nucleinsäuren, Oligonucleotiden, biologischen Zellen, Antigenen, Antikörpern und Haptenen besteht.

23. Verfahren nach mindestens einem der Ansprüche 19 bis 22, wobei das Teilchen Rinderserumalbumin umfaßt, das mit dem funktionalisierten Polymer gekoppelt ist.

24. Verfahren zur Bestimmung der Anwesenheit oder Konzentration von spezifischen Nucleinsäuresequenzen in Nucleinsäure-Zielmolekülen, das folgende Schritte aufweist:
Kontaktieren der magnetischen Teilchen gemäß Anspruch 3 oder 5, bei denen eine zur Nucleinsäuresequenz des Zielmoleküls komplementäre Nucleinsäure mit dem funktionalisierten Polymer gekoppelt ist, mit einer Fluidprobe zur Bildung einer Suspension;
Inkubieren der Suspension unter Hybridisierungsbedingungen für eine ausreichende Zeitdauer, die die Hybridisierung ermöglicht;
Abtrennen der magnetischen Teilchen von der Suspension;
Hinzusetzen einer zweiten, markierten Nucleinsäureseguenz, die zur Nucleinsäuresequenz des Zielmoleküls komplementär ist;
Inkubieren der Suspension unter Hybridisierungsbedingungen für eine ausreichende Zeitdauer, die die Hybridisierung ermöglicht;
Abtrennen der magnetischen Teilchen von der Suspension; und
Feststellen einer Doppelstrang-Bildung an den magnetischen Teilchen mit Hilfe der Markierung.

25. Verfahren nach Anspruch 24,
wobei die markierte Nucleinsäuresequenz, die zur Nucleinsäuresequenz des Zielmoleküls komplementär ist, mit Biotin markiert wird und die Markierung durch ein markiertes Avidin verstärkt wird.

26. Verfahren nach Anspruch 24,
wobei die zweite, markierte Nucleinsäuresequenz mit β-D-Galactosidase markiert wird und die Menge der mit dem magnetischen Teilchen gekoppelten, markierten Nucleinsäuresequenz unter Verwendung des Substrates 4-Methylumbelliferyl-β-galactopyranosid und eines Fluoreszenz-Analysegerätes bestimmt wird.

27. Verfahren nach Anspruch 26,
wobei das Fluoreszenz-Analysegerät ein Erregungsfilter von etwa 365 nm und ein Emissionfilter von etwa 450 nm besitzt.

28. Verfahren zur Isolierung einer Biosubstanz, das folgende Schritte aufweist:
Kontaktieren magnetischer Teilchen gemäß Anspruch 3 oder 5, die einen für die Biosubstanz spezifischen Liganden tragen;
Inkubieren der Suspension bis sich eine ausreichende Menge Biosubstanz mit dem Liganden umgesetzt hat;
Abtrennen der magnetischen Teilchen von der Suspension;
Abtrennen der magnetischen Teilchen von der Biosubstanz; und
Erhalten der im wesentlichen reinen Biosubstanz.

29. Verfahren zur Entfernung nicht erwünschter Biosubstanz, das folgende Schritte aufweist:
Kontaktieren magnetischer Teilchen gemäß Anspruch 3 oder 5, die einen für die Biosubstanz spezifischen Liganden tragen;
Inkubieren der Suspension bis sich eine ausreichende Menge Biosubstanz mit dem Liganden umgesetzt hat;
Abtrennen der magnetischen Teilchen von der Suspension; und
Erhalten einer von nicht erwünschter Biosubstanz freien Suspension.

30. Verfahren nach Anspruch 28 oder 29,
wobei die Biosubstanz ein Protein ist.

31. Verfahren nach Anspruch 28 oder 29,
wobei die Biosubstanz biologische Zellen sind.

32. Verfahren nach Anspruch 31,
wobei die Biosubstanz Knochenmarkzellen umfaßt.

33. Verfahren zur industriellen Abfallreinigung, umfassend das Entfernen von unerwünschten Substanzen aus industriellem Abfall unter Verwendung der magnetischen Teilchen gemäß Anspruch 3 oder 5.

## Revendications

1. Particules magnétiques monodispersées ayant une répartition granulométrique uniforme et une teneur uniforme en substances magnétiques, comprenant :
un polymère de coeur intérieur particulaire, à même d'adsorber un monomère ; et
une combinaison d'un oxyde métallique à réponse magnétique et d'un polymère, que l'on prépare en polymérisant un mélange d'un oxyde métallique et d'un monomère en présence d'un amorceur dans le mélange, ce polymère étant constitué de monomères à même d'être adsorbés par les particules du polymère de coeur intérieur, cette combinaison d'un oxyde métallique et d'un polymère recouvrant d'une manière uniforme les particules de coeur intérieur, les particules magnétiques ayant une répartition granulométrique uniforme, une teneur uniforme en substances magnétiques et étant monodispersées en solution.

2. Particules magnétiques selon la revendication 1, qui comprennent un enrobage polymère extérieur, qui recouvre la combinaison de l'oxyde métallique à réponse magnétique et du polymère.

3. Particules magnétiques selon la revendication 2, qui comprennent une couche d'un polymère fonctionnalisé recouvrant l'enrobage polymère extérieur.

4. Particules magnétiques selon les revendications 2 ou 3, dans lesquelles l'enrobage polymère extérieur comprend un polymère choisi parmi l'ensemble comprenant le polystyrène, le polystyrène réticulé ou le polystyrène fonctionnalisé.

5. Particules magnétiques selon la revendication 1, qui comprennent une couche d'un polymère fonctionnalisé recouvrant la combinaison de l'oxyde métallique et du polymère, ces particules magnétiques ayant une répartition granulométrique uniforme, une teneur uniforme en substances magnétiques et étant monodispersées en solution.

6. Particules magnétiques selon les revendications 3, 4 (quand elle dépend de la revendication 3) ou 5, dans lesquelles le polymère fonctionnalisé est un composé qui apporte des groupes fonctionnels carboxyle, amino ou hydroxyle, pour couplage à un matériau biologique.

7. Particules magnétiques selon les revendications 3, 4 (quand elle dépend de la revendication 3) ou 5, dans lesquelles le polymère fonctionnalisé est couplé à un matériau biologique.

8. Particules selon l'une quelconque des revendications ci-dessus, dans lesquelles les particules de coeur comprennent du polystyrène ou du polystyrène réticulé.

9. Particules selon l'une quelconque des revendications ci-dessus, dans lesquelles la granulométrie des particules de coeur est comprise entre environ 1 et 100 µm.

10. Particules selon l'une quelconque des revendications précédentes, possédant au moins un enrobage supplémentaire constitué d'une combinaison d'un oxyde métallique et d'un polymère, qui recouvre uniformément le premier enrobage constitué d'une combinaison d'un oxyde métallique et d'un polymère.

11. Particules selon l'une quelconque des revendications ci-dessus, dans lesquelles l'oxyde métallique à réponse magnétique de l'enrobage constituant au moins l'un des enrobages ci-dessus comprend des particules ayant un diamètre d'environ 1 µm ou moins.

12. Particules selon l'une quelconque des revendications ci-dessus, dans lesquelles au moins l'une des couches métalliques à réponse paramagnétique comprend un oxyde métallique superparamagnétique, paramagnétique ou ferromagnétique.

13. Particules selon l'une quelconque des revendications ci-dessus, dans lesquelles au moins l'une des combinaisons d'un oxyde métallique à réponse magnétique et d'un polymère comprend du polystyrène, du polystyrène réticulé ou un polymère fonctionnalisé.

14. Particules selon l'une quelconque des revendications 1 à 4, dans lesquelles l'oxyde métallique d'au moins l'un des enrobages est constitué de sels de métaux de transition.

15. Procédé pour fabriquer des particules polymères à réponse magnétique ayant des particules de coeur polymères uniformément recouvertes par un enrobage constitué d'une combinaison d'un oxyde métallique et d'un polymère, qui consiste :
à collecter des particules d'oxyde métallique ayant un diamètre d'environ 1 µm ou moins ;
à mélanger ces particules d'oxyde métallique avec un monomère pour former un enrobage constitué d'une combinaison d'un oxyde métallique et d'un polymère ; et
à enrober en présence d'un amorceur des particules de coeur polymères, avec l'enrobage constitué de la combinaison de l'oxyde métallique et du polymère.

16. Procédé selon la revendication 15, dans lequel on produit l'oxyde métallique par les étapes consistant :
(a) à chauffer et faire précipiter un mélange de sels de métaux de transition divalents et trivalents avec de l'hydroxyde de sodium ;
(b) à laver l'oxyde métallique précipité jusqu'à pH neutre ;
(c) à remettre en suspension l'oxyde métallique dans de l'eau déminéralisée ;
(d) à agiter l'oxyde métallique pour casser l'agrégat de cristaux d'oxyde métallique ; et
(e) à procéder à une centrifugation à faible vitesse pour diminuer la granulométrie des cristaux d'oxyde métallique.

17. Procédé selon l'une quelconque des revendications 15 ou 16, dans lequel les particules constituées d'une combinaison de l'oxyde métallique et du polymère sont mélangées à un ou plusieurs monomères pour former un enrobage constitué d'une combinaison d'un oxyde métallique et d'un polymère, ce monomère étant choisi parmi l'ensemble comprenant le styrène, le divinylbenzène, le méthacrylate de méthyle, le méthacrylate de glycidyle et d'autres monomères oléfiniques.

18. Procédé selon l'une quelconque des revendications 15 à 17, dans lequel les particules de coeur polymère ont une granulométrie de 1 à 100 µm.

19. Procédé pour déterminer la présence ou la concentration d'un analyte, qui consiste :
à mettre en contact avec un échantillon de fluide pour former une suspension des particules magnétiques selon les revendications 3 ou 5 ayant un ligand spécifique de l'analyte, fixé au polymère fonctionnalisé;
à incuber cette suspension jusqu'à ce qu'une quantité suffisante d'analyte ait réagi avec le ligand spécifique ; à séparer de la suspension les particules magnétiques ;
à ajouter un deuxième ligand marqué, spécifique de l'analyte, aux particules magnétiques séparées ;
à incuber la suspension jusqu'à qu'une quantité suffisante d'analyte ait réagi avec le deuxième ligand marqué spécifique de l'analyte ;
à séparer de la suspension les particules magnétiques ;
à mesurer la quantité de ligand marqué associée aux particules magnétiques ;
à établir une relation entre la quantité mesurée du ligand marqué et la quantité mesurée de l'analyte dans un échantillon témoin.

20. Procédé selon la revendication 19, dans lequel le deuxième ligand est marqué à la β-D-galactosidase, et la quantité du ligand marqué associée à la particule magnétique est mesurée par utilisation du substrat 4-méthylumbelliféryl-β-galactopyrannoside et d'un analyseur à fluorescence.

21. Procédé selon la revendication 20, dans lequel l'analyseur à fluorescence possède un filtre d'excitation à environ 365 nm et un filtre d'émission à environ 450 nm.

22. Procédé selon l'une quelconque des revendications 19 à 21, dans lequel l'analyte est choisi parmi l'ensemble comprenant les enzymes, les hormones, les peptides, les vitamines, les acides nucléiques, les oligonucléotides, les cellules biologiques, les antigènes, les anticorps et les haptènes.

23. Procédé selon l'une quelconque des revendications 19 à 22, dans lequel les particules comprennent de l'albumine de sérum de boeuf fixée au polymère fonctionnalisé.

24. Procédé pour déterminer la présence ou la concentration de séquences spécifiques d'acides nucléiques dans des molécules cibles d'acides nucléiques, qui consiste :
à mettre en contact des particules magnétiques selon les revendications 3 ou 5, ayant un acide nucléique complémentaire de la séquence d'acides nucléiques de la molécule cible, fixée au polymère fonctionnalisé, avec un échantillon fluide pour former une suspension ;
à incuber la suspension dans des conditions d'hybridation pendant un laps de temps suffisant pour permettre l'hybridation ;
à séparer de la suspension les particules magnétiques ;
à ajouter une deuxième séquence d'acides nucléiques complémentaire de la séquence d'acides nucléiques de la molécule cible ;
à incuber la suspension dans des conditions d'hybridation pendant un laps de temps suffisant pour permettre l'hybridation ;
à séparer de la suspension les particules magnétiques ; et
à détecter la formation d'un duplex sur les particules magnétiques à l'aide du marqueur.

25. Procédé selon la revendication 24, dans lequel la séquence d'acides nucléiques marquée complémentaire de la séquence d'acides nucléiques de la molécule cible est marquée à la biotine, ce marqueur étant amplifié par une avidine marquée.

26. Procédé selon la revendication 24, dans lequel la deuxième séquence d'acides nucléiques marqués est marquée à la β-D-galactosidase, et la quantité de la séquence d'acides nucléiques marqués associée à la particule magnétique est mesurée à l'aide du substrat 4-méthylumbelliféryl-β-galactopyrannoside et d'un analyseur à fluorescence.

27. Procédé selon la revendication 26, dans lequel l'analyse à fluorescence possède un filtre d'excitation à environ 365 nm et un filtre d'émission à environ 450 nm.

28. Procédé pour isoler une biosubstance, qui consiste :
à mettre en contact les particules magnétiques selon les revendications 3 ou 5 possédant un ligand spécifique de la biosubstance ;
à incuber la suspension jusqu'à ce qu'une quantité suffisante de la biosubstance ait réagi avec le ligand ;
à séparer de la suspension les particules magnétiques ;
à séparer de la biosubstance les particules magnétiques ; et
à obtenir une biosubstance essentiellement pure.

29. Procédé pour éliminer une biosubstance indésirable, qui consiste :
à mettre en contact des particules magnétiques selon les revendications 3 ou 5 possédant un ligand spécifique de la biosubstance ;
à incuber la suspension jusqu'à ce qu'une quantité suffisante de biosubstance ait réagi avec le ligand ;
à séparer de la suspension les particules magnétiques ; et
à obtenir une suspension exempte de la biosubstance indésirable.

30. Procédé selon les revendications 28 ou 29, dans lequel la biosubstance est une protéine.

31. Procédé selon les revendications 28 ou 29, dans lequel la biosubstance est constituée de cellules biologiques.

32. Procédé selon la revendication 31, dans lequel la biosubstance comprend des cellules médullaires.

33. Procédé de purification industrielle des déchets, qui consiste à éliminer des déchets industriels les substances indésirables par utilisation des particules magnétiques selon les revendications 3 ou 5.
